# EUROPEAN PATENT SPECIFICATION

(11) **EP 4 452 932 B1**
(45) Date of publication and mention of the grant of the patent: **01.10.2025**
(21) Application number: 22829621.6
(22) Date of filing: 19.12.2022
(51) Int. Cl.: C07C 273/04, B01D 3/00

(54) **THERMAL STRIPPING UREA PRODUCTION**
HARNSTOFFHERSTELLUNG DURCH THERMISCHES STRIPPING
PRODUCTION D'URÉE PAR DÉCAPAGE THERMIQUE

(30) Priority: 20.12.2021 EP 21216056
(43) Date of publication of application: 30.10.2024
(73) Proprietor: Stamicarbon B.V., 6135 KW Sittard (NL)
(72) Inventor: VAN DEN TILLAART, Johan Albert Arno, 6135 KW Sittard (NL); MOSTERT, Eelco, 6135 KW Sittard (NL)
(74) Representative: V.O.
(86) International application number: PCT/NL2022/050732
(87) International publication number: WO 2023/121443

(56) References cited:
- EP-A1- 2 128 129

## Description

### Field

The invention pertains to the production of urea in a urea plant of the thermal stripping type (also known as self-stripping type).

### Introduction

An example urea process of the thermal stripping type is illustrated in Ullmann's Encyclopaedia of Industrial Chemistry, Chapter Urea, 2010, Figure 25. In the illustrated process, the high pressure synthesis section comprises a vertical reactor, a thermal stripper, a high pressure carbamate condenser of the kettle type, a carbamate separator, and an ammonia-driven liquid-liquid ejector. The high pressure carbamate condenser is a shell-and-tube heat exchanger with a U-shaped tube bundle wherein gas from the stripper is received in the tubes and steam is raised on the shell side.

US4082797A describes a carbamate condensation zone for a urea plant composed of a horizontal tube bundle which is placed under the static pressure of a liquid head. GB1184004A describes a thermal stripper for a urea plant.

EP2128129 describes a method for revamping a conventional self-stripping urea plant , wherein the conventional horizontal shell-and-tube carbamate condenser is replaced with a vertical submerged unit. It is mentioned that the flow lines of gaseous ammonia and carbon dioxide from the stripper and recycled carbamate solution are modified so as to feed them to tubes of the new vertical submerged condenser.

There remains a desire for improved urea plants of the thermal stripping type. There is also a desire for methods of modifying existing urea plants of the thermal stripping type to increase capacity and/or improve operation.

### Summary

The invention pertains in a first aspect to a urea production plant as defined in claim 1.

The invention also pertains to a urea production process carried out in the urea production plant according the invention, the process comprising: reacting NH₃ and CO₂ in the reactor giving a urea synthesis stream; supplying at least a liquid part of the synthesis stream to the stripper; supplying gas from the stripper to the shell space of the HP carbamate condenser; and condensing said gas in said shell space of the HP carbamate condenser.

The invention also pertains to a method of modifying an existing urea production plant as defined in claim 20.

### Brief description of the drawings

Figure 1 schematically illustrates an example urea production process scheme according to the invention.
Figure 2 schematically illustrates an example HP carbamate condenser (pool condenser) that can be used in the invention.
Figure 3 schematically illustrates an example HP carbamate condenser (pool reactor) that can be used in the invention.
Figure 4 schematically illustrates an example thermal stripper that can be used in the invention.
Figure 5 schematically illustrates an example kettle-type boiler as used in existing plants (not according to the invention).

Any embodiments illustrated in the figures are examples only and do not limit the invention.

### Detailed description

The invention pertains to a urea production process, to a plant of the thermal stripping type, and to a method of modifying an existing plant of the thermal stripping type into an inventive plant of the thermal stripping type. The urea production process is carried out in the plant according to the invention. The method of modifying an existing plant gives a urea production plant according to the invention.

The invention can be used both for newly built urea plants of the thermal stripping type ('grassroots') and for modifying existing urea plants of the thermal stripping type ('revamp'), as well as for other types of modifications of existing plants.

The plant comprises a high pressure (HP) synthesis section comprising a reactor, a stripper and a condenser, all operating at high pressure. The synthesis section furthermore typically comprises a carbamate separator and an ejector also operating at high pressure.

The reactor has an outlet for synthesis fluid connected to an inlet of the stripper, for flow of at least liquid from an outlet of the reactor to the stripper.

The reactor is for instance a vertical reactor with one or more inlets at the bottom and an outlet at the top and e.g. trays. The reactor typically has an inlet for CO₂ feed at the bottom and an inlet for a stream comprising NH₃ feed and carbamate.

The stripper is a shell-and-tube heat exchanger with urea solution to be stripped in the tubes, as a falling film, and with heating fluid, in particular steam, in the shell. The stripper has an outlet for stripped urea solution at the bottom and an outlet for gas at the top, and an inlet for a fluid stream comprising liquid comprising urea from the reactor at the top. In some embodiments, the fluid stream from the reactor contains both gas and liquid. In some embodiments, the plant comprises a HP gas/liquid separator in the reactor or in the fluid flow line between the reactor and the stripper top and liquid from the separator is supplied to the stripper top and gas from the separator is supplied e.g. also to the top of the stripper or e.g. to the bottom of the stripper (tube side) or to another unit.

The stripper tubes are e.g. Zr or Ti tubes or bimetallic tubes. The bimetallic tubes for example each comprise or consist of two coaxial tubes; for example an external tube made of steel, e.g. 25Cr-22Ni-2Mo, and an internal tube made of e.g. zirconium. Preferably the bimetallic tubes comprise an external steel tube and an internal tube of e.g. Zr. The internal and external tube of a bimetallic tube preferably have a metallurgical bond.

Other types of tube materials for the stripper tubes are also possible, such as duplex ferritic-austenitic stainless steels. Suitable duplex stainless steel for the stripper tubes include for example the steel available as Safurex^{®} steel (Safurex^{®} Star) and having composition 29Cr-6.5Ni-2Mo-N, which steel is also designated by ASME Code 2295-3 and by UNS S32906, or e.g. the steel available as DP28W^{™} steel and having composition 27Cr-7.6Ni-1Mo-2.3W-N, which is also designated by ASME Code 2496-1 and by UNS S32808. The duplex steel for the stripper tubes has for instance the composition (% by weight, wt.%): C: max. 0.05, Si: max. 0.8, Mn: 0.3 - 4.0, Cr: 28 - 35, Ni: 3 - 10, Mo: 1.0 - 4.0, N: 0.2 - 0.6, Cu: max. 1.0 W: max. 2.0 S: max. 0.01 Ce: 0 - 0.2, balance Fe and (unavoidable) impurities. Preferably, the ferrite content is 30-70% by volume and more preferably 30-55%. More preferably, the steel contains (wt.%): C max. 0.02, max. 0.5 Si, Cr 29 to 33, Mo 1.0 to 2.0, N 0.36 to 0.55, Mn 0.3 to 1.0, balance Fe and impurities. Also suitable is a duplex stainless steel having the composition (wt.%): C max 0.030; Si max 0.8; Mn max 2.0; Cr 29.0 to 31.0; Ni 5.0 to 9.0; Mo less than 4.0; W less than 4.0; N 0.25-0.45; Cu max 2.0; S max 0.02; P max 0.03; balance Fe and unavoidable occurring impurities; and wherein the content of Mo+W is greater than 3.0 but less than 5.0 (wt.%), furthermore preferably with a steel composition as described in WO 2017/014632. These steel compositions may also be used for the external tube of a bimetallic tube.

The high pressure (HP) carbamate condenser is configured for condensing gas from the stripper top, which contains NH₃ and CO₂, into ammonium carbamate, in a condensation space of the condenser. Thereby a liquid phase is formed in the condensation space of the condenser which contains ammonium carbamate, and in the present invention also urea and water. The condenser also receives carbamate recycle solution, in particular from a medium pressure (MP) recovery of the urea plant, in the condensation space.

The HP carbamate condenser is a shell-and-tube heat exchanger comprising a tube bundle, a shell, and a shell space (i.e. a shell side space). In the invention, the condensation space is the shell space of the condenser. Accordingly, carbamate condensation is conducted in the shell space of the condenser. This provides for a difference with the kettle-type boilers used in prior art urea plants of the thermal stripping type.

The condenser is a heat exchanger using indirect heat exchange with a cooling fluid, e.g. with boiler feed water as the cooling fluid. In the invention, the cooling fluid is supplied to the tubes.

In embodiments wherein the plant comprises a separate reactor and condenser, the plant comprises a liquid flow line for supplying carbamate-containing liquid formed in the shell space of the HP carbamate condenser to the reactor.

Thereby the condenser in the inventive plant differs from a kettle-type condenser as used e.g. in Ullmann's Encyclopaedia of Industrial Chemistry, Chapter Urea, 2010, Figure 25.

In example embodiments of the inventive plant, the shell space comprises an outlet for a fluid stream containing both gas and liquid, which outlet is connected to a HP gas/liquid separator (carbamate separator) having a gas outlet and a liquid outlet. The liquid outlet of the separator is connected, typically through an ejector, to an inlet of the reactor, to supply a liquid containing carbamate, urea and water to the reactor. The gas outlet is for instance connected with the MP recovery section, e.g. to supply the gas directly or indirectly to a medium pressure (MP) carbamate condenser.

In some embodiments of the invention, the condenser has a gas outlet and a separate liquid outlet both from the shell space (shell side gas outlet and separate shell side liquid outlet). The shell side liquid outlet is for example connected indirectly with the reactor through the high pressure (HP) ejector. In some embodiments, the gas from the shell side gas outlet is supplied to the MP recovery section, in particular directly or indirectly to an MP carbamate condenser. Thereby the additional space in the shell side, compared to condensation in the tubes, may advantageously also allow for gas/liquid separation already in the condenser, e.g. in the condenser vessel. This may obviate the need for a separate carbamate separator. The gas/liquid separation may e.g. be provided in the top of the condenser vessel or e.g. using a baffle configured as overflow weir.

The ejector typically uses the high pressure (HP) ammonia feed as motive fluid, in particular compressed liquid NH₃ fed to the reactor.

Alternatively or additionally, a pump is for instance used for transporting liquid from the condenser, e.g. through the carbamate separator, to the reactor.

The synthesis section may comprise two (or more) HP carbamate condensers, e.g. two HP carbamate condensers in series or in parallel. One or both of the two HP carbamate condenser is e.g. a shell-and-tube heat exchanger configured for condensation in the shell as described.

The process is carried out for instance with a reactor pressure in the range 120 - 200 bar, e.g. 140 - 165 bar. The process is carried out typically with a reactor outlet N/C ratio of 2.8 - 4.0, preferably 3.2 - 3.6, wherein the N/C ratio refers to the theoretical initial mixture. The reactor outlet temperature is for instance in the range 180 - 200°C, preferably 185 - 190°C. The reactor CO₂ conversion is for example above 50%, for instance above 60%.

The high reactor outlet N/C ratio of preferably 3.2 - 3.6 contributes to the stripping effect in the thermal stripper. The stripper pressure is for instance in the range 145 - 150 bar in the tubes. Typically, the stripper is operated at a pressure lower than the reactor, e.g. at least 2 bar lower or at least 5 bar lower, typically max 10 bar lower. Typically the condenser is operated at the same pressure as the stripper, e.g. in the range 145-150 bar.

The stripper inlet (top) temperature is e.g. 185 - 195°C. The stripper outlet temperature is e.g. at least 200°C, preferably e.g. at least 205°C, for example less than 220°C. The NH₃ content in the stripper bottom effluent is e.g. 20 - 30 wt.%, e.g. 20-25 wt.%.

The stripped urea solution is supplied to an MP recovery section. A urea solution from the MP recovery section is supplied to an low pressure (LP) recovery section. A urea solution from the LP recovery section can be supplied, for instance, to an evaporation section to form a urea melt. The urea melt is supplied, for instance, to a finishing section to form a solid urea product. The finishing section is for instance a granulator or a prilling tower. The urea melt can for instance also be used for the production of melamine. The urea solution can for instance also be used for the production of liquid fertilizer such as urea ammonium nitrate (UAN). The urea can also be used e.g. for NOx abatement, in particular for making Diesel Exhaust Fluid and Diesel Exhaust Fluid precursor products.

The MP recovery section (or recirculation section) comprises a medium pressure (MP) decomposer receiving stripped urea solution, optionally through a rectifier. The MP decomposer is a shell-and-tube heat exchanger typically using steam as heating fluid. The MP decomposer has a gas outlet connected to an MP carbamate condensation section. The MP carbamate condensation section for instance comprises a first MP carbamate condenser for carbamate condensation in indirect heat exchange with urea solution to be heated in a condenser/preheater (e.g. a shell-and-tube heat exchanger with urea solution to be heated in the tubes, carbamate condensation at MP in the shell). The MP carbamate condensation section for example comprises a second MP carbamate condenser for and with second carbamate condensation using cooling water. The second MP carbamate condenser for instance receives non-condensed gas from the first MP carbamate condenser. A fluid stream from the MP carbamate condenser(s) is supplied to an ammonia-carbamate separation column. For instance liquid ammonia reflux is applied in the separation column. The separation column has an outlet for gas connected to an ammonia condenser, which ammonia condenser is connected to an ammonia receiver, wherein the ammonia receiver also receives a part or all of the NH₃ feed. The ammonia receiver has a liquid outlet to an ammonia pump which is used for pumping the ammonia in part to the separation column and in part to a high pressure (HP) ammonia pump connected to an ammonia flow line to the HP ejector. Thereby ammonia is recycled as ammonia stream from the MP recovery section to the HP reactor, said stream also containing a part or all of the NH₃ feed, and said ammonia stream being the motive fluid for the HP ejector. The separation column also has a bottom outlet for liquid connected to a HP carbamate pump for supplying recycle carbamate solution to the HP carbamate condenser.

In the invention, the MP recovery section has separate outlets for ammonia and for carbamate solution which are recycled separately to the HP synthesis section.

The LP recovery section comprises a low pressure (LP) decomposer, receiving urea solution from the MP recovery section, in particular from the MP decomposer, and having an outlet for urea solution typically connected to the urea solution preheater, and an outlet for gas connected to aa low pressure (LP) carbamate condenser. A low pressure (LP) aqueous carbamate solution from the LP recovery section, in particular from the LP carbamate condenser, is supplied to the MP recovery section, for example to the shell of the preheater where carbamate is condensed. The water fraction in the LP carbamate recycle solution can be used to prevent crystallization of carbamate in the MP condensation section.

The HP carbamate condenser in the plant of the invention is a shell-and-tube heat exchanger configured for condensation in the shell, i.e. in the shell-side space, and for having cooling fluid in the tubes. For instance the cooling fluid is boiler feed water and steam is raised in the tubes. The condenser has an inlet for gas from the stripper to be condensed into the shell and an outlet from the shell for a fluid stream comprising a liquid comprising carbamate, water, and urea.

By performing the carbamate condensation in the shell space, a relatively longer residence time can be provided compared to condensers with condensation in the tubes. This advantageously increases the synthesis efficiency, i.e. the conversion into urea.

Moreover, the efficiency of the condensation is advantageously higher due to the higher effective heat transfer coefficient. Furthermore the liquid outlet temperature (condensate) is advantageously significantly higher due to the higher concentration of urea in the liquid. The higher urea content increases the condensation temperature. This higher condensation temperature advantageously allows e.g. for raising steam with higher pressure in the tubes and/or e.g. for using a smaller heat exchanging surface, i.e. smaller equipment size. Moreover, due to the formation of urea in the condenser a part of the heat is used internally and does not need to be removed from the condenser by heat transfer through the tubes. These effects allow for raising higher pressure steam, a smaller tube bundle or higher synthesis capacity, or a combination of these advantages. The higher liquid outlet temperature may also increase the temperature of the vertical reactor thereby improving CO₂ conversion and urea yield in the vertical reactor and/or allowing for a lower pressure of the vertical reactor which may contribute to improved stripping in the thermal stripper.

The HP carbamate condenser is, in the shell space, a gas-agitated vessel wherein the gases CO₂ and NH₃ condense in an exothermic reaction into carbamate around a bundle of tubes in the shell space, with the vessel providing a retention time enabling the condensed carbamate to dehydrate partially into urea and water in the liquid phase. The dehydration reaction is endothermic and slower than the carbamate condensation reaction. The liquid phase in the shell space of the condenser is thoroughly agitated by the gases from the stripper. In operation, a pool of liquid is present in the shell space submerging the tube bundle. The residence time provided by the shell space volume may allow the reaction of ammonium carbamate to urea and water to proceed to an advantageous high degree. The formation of gas bubbles in the shell space e.g. using a sparger, ensures a high degree of turbulence and provides a large area for mass and heat transfer between the gas to be condensed and the liquid in the shell space.

In some embodiments, the reaction of ammonium carbamate to urea and water proceeds to such an extent that the liquid from the shell space outlet contains e.g. 2 - 30 wt.% urea, for example at least 5 wt.%, or at least 10 wt.% urea, and/or up to 25 wt.% urea or up to 20 wt.% urea, e.g. 5 - 25 wt.% urea, 10 - 25 wt.% urea, or 10 - 20 wt.% urea.

The urea synthesis solution, e.g. the liquid from the reactor, contains e.g. 30 - 40 wt.% urea.

The urea is formed in the shell space of the condenser by virtue of the relatively long residence time in the relatively large volume of the shell space in the condenser.

The condensation temperature on the shell side in some embodiments can be relatively high, e.g. at least 165°C or at least 170°C, as a result of the formation of high boiling components (urea and water). Preferably, the liquid outlet temperature of the shell space of the condenser is at least 165°C, such as at least 170°C.

The HP carbamate condenser preferably has a horizontal tube bundle, e.g. a U-shaped tube bundle with horizontal tube legs. The horizontal submerged carbamate condenser advantageously has a relatively high heat transfer coefficient.

The HP carbamate condenser for instance comprises a sparger (gas divider) for distributing gas to be condensed in the shell. The sparger is arranged horizontally, i.e. for horizontally distributing gas to be condensed in the shell, and is used in combination with a horizontal tube bundle and condensation in the shell space of the condenser. The sparger can contribute to enhanced mixing of the process fluids, over the whole volume of the shell space, in particular gas/liquid mixing.

The sparger is a device used to uniformly introduce a fluid, i.e. the gas from the stripper, into a second body of fluid, i.e. the process fluid in the shell space. The sparger is for instance constructed as a horizontal tube with holes. The sparger is arranged at the bottom of the shell space, in particular below the tube bundle. The sparger is for example a horizontal tube with side branches with holes on the top configured for distributing the gas, in the shell space, over the length and optionally also width of the shell space of the condenser. The sparger advantageously contributes to achieving optimal conditions in the shell space.

The gaseous stream leaving the stripper is distributed within the condenser via the sparger and is mixed with carbamate solution in the shell space of the condenser. The carbamate solution is e.g. supplied from the MP recovery section. The mixing of gas/liquid within the shell space of the condenser advantageously obviates the need for external mixing equipment.

The shell space is for instance provided with vertical deflector plates arranged parallel to the tube bundle and possibly baffles.

For instance a pool condenser is used, e.g. as described in Nitrogen No. 222, July August 1996, pp.29-31, or e.g. as illustrated in Fig. 18 of Ullmann's Encyclopaedia of Industrial Chemistry, Chapter Urea, 2010 (for a CO₂-stripping type urea plant). For instance the HP carbamate condenser is a pool condenser comprising a horizontal U-shaped tube bundle which extends over essentially the entire length of the shell space. The length direction is parallel to the legs of the U-shaped tube bundle of the pool condenser. The pool condenser may have a single outlet from the shell space or separate outlets for gas and for liquid from the shell space.

Optionally a pool rector is used, e.g. as described in US 5767313, or e.g. as illustrated in Fig. 19 of Ullmann's Encyclopaedia of Industrial Chemistry, Chapter Urea, 2010 (for a CO₂-stripping type urea plant).

In some embodiments, e.g. with a pool reactor, the HP carbamate condenser is provided as a vessel comprising a horizontal U-shaped tube bundle and a shell space comprising a condensation zone and a reaction zone. For instance, the tube bundle extends over a part, e.g. 10 - 70 %, such as 20 - 50 %, of the length of the shell space, thereby providing the condensation zone of the vessel where the tube bundle is located, and the reaction zone. Herein the legs of the U-shaped tubes are arranged horizontally in the length of the vessel. The reaction zone is located, horizontally, between the bend the U-shaped tube bundle and the liquid outlet of the shell space. The vessel comprises a tube sheet. The condensation zone is located between the tube sheet and the bend of the U-shaped tube bundle. Such a configuration is referred to as 'pool reactor' herein. The pool reactor has a gas outlet from the top of the shell space. The pool reactor typically contains baffles in the shell space. The baffles are arranged perpendicular to the tube bundle and are provided e.g. with openings or gaps and can be used e.g. to provide for compartments over the length of the pool reactor. Typically in a pool reactor the sparger extends horizontally in the length of the vessel beyond the U-shaped tube bundle into the reaction zone. The sparger typically also extends horizontally in the condensation zone. Typically a part of the sparger is provided below the U-shaped tube bundle.

Generally, the pool reactor has a liquid outlet for urea solution connected, typically through a liquid-liquid ejector, to a vertical urea reactor, e.g. an after-reactor.

The plant further typically comprises a steam drum receiving fluid from the outlet of the tube bundle, said fluid comprising steam and entrained water droplets. The preferred steam drum is provided in connection with raising steam in the tubes of the HP carbamate condenser.

The steam drum is used for purifying the steam by removal of the water droplets. The steam drum is typically a gas/liquid separator operating on gravity. The steam drum is typically a vertical vessel with an inlet at the side, an outlet for liquid at the bottom and a gas outlet at the top. Dry steam from the gas outlet of the steam drum can be used further in the process e.g. for heating. The steam drum can be connected directly to the condenser, in particular to the outlet header of the tube bundle, or through a duct. The liquid from the steam drum is typically recycled to the inlet header of the tube bundle of the condenser. The steam drum comprises e.g. a mist eliminator. The mist eliminator is e.g. a knitted wire mesh pad mist eliminator between the inlet and gas outlet.

The plant may comprise an additional HP carbamate condenser which can be arranged in series or in parallel (for the process stream) to the HP carbamate condenser. The additional HP carbamate condenser can e.g. be a kettle-type condenser as present e.g. in an existing urea plant. In embodiments wherein the HP carbamate condenser and the additional HP carbamate condenser are arranged in series (for the gas to be condensed), the HP carbamate condenser may be placed upstream or downstream of the additional HP carbamate condenser.

The HP carbamate condenser is placed e.g. at a raised position and level with the steam drum. The condenser may be placed e.g. above the optionally used additional HP carbamate condenser. This may be advantageous in a revamp of an existing thermal stripping urea plant.

In an alternative embodiment, the HP carbamate condenser has a vertical tube bundle, for instance a U-shaped tube bundle with vertical legs of the tubes. For example the condenser is provided with a horizontal tube sheet, an inlet header for cooling fluid and an outlet header for heated cooling fluid (e.g. steam), all at the bottom of the condenser.

Preferably, carbamate recycle solution from the MP recovery section is introduced into the HP carbamate condenser separately from the gas mixture from the stripper. The condenser comprises for example a liquid distributor or one or more nozzles in the shell space for introducing the carbamate recycle solution into the shell space.

In operation, the tube bundle is submerged by process medium on the shell side. Typically, low pressure (LP) steam is generated in the tubes. The steam pressure is e.g. 2 - 9 bar, advantageously for instance 5.0 - 9.0 bar or e.g. 5.0 - 6.0 bar.

The LP steam formed in the HP carbamate condenser is for instance used as heating fluid in the MP decomposer. Other uses are also possible.

It is noted that in a typical prior art thermal stripping urea plant, the LP steam needed in the MP decomposer is in part obtained from shell side of the HP carbamate condenser and in part from medium pressure (MP) steam. Accordingly, the higher pressure LP steam obtained in embodiments of the present invention may advantageously provide for a reduction of the MP steam consumption of the urea plant.

The shell (i.e. the vessel wall) of the HP carbamate condenser is typically provided with a lining of urea grade corrosion resistant steel, e.g. with a lining in duplex ferritic-austenitic stainless steel, on the internal surface which is exposed to the shell space, because in operation corrosive process medium comprising carbamate is present in the shell space. The shell wall is e.g. made of carbon steel and able to withstand high pressures in the shell space, e.g. at least 140 bar.

The tubes are made e.g. of corrosion-resistant steel, e.g. of duplex ferritic-austenitic stainless steel. In embodiments with a reaction zone in the HP carbamate condenser, e.g. embodiments using a pool reactor, the amount of urea grade steel required for the reaction zone may be relatively low compared to providing the same reaction volume in tubes.

The condenser comprises typically a tubesheet and typically the legs of the tubes are connected to the tubesheet, optionally through sleeves, or other joining means, and. e.g. using internal bore welding. The tubesheet is e.g. provided, on the side exposed to the shell space, with overlay welding and/or lining of urea grade corrosion resistant steel, e.g. with a duplex stainless steel lining.

Internals in the shell space are preferably constructed of urea grade corrosion resistant steel, e.g. duplex stainless steel.

The HP carbamate condenser is placed e.g. at ground level or at elevated level, e.g. at a level up to 10 m above ground level.

Optionally, a part of the CO₂ feed is supplied to the HP carbamate condenser, directly or indirectly, e.g. via a high pressure (HP) stripper, or using a combination of both directly and indirectly feeding to the HP carbamate condenser.

Optionally, a part of the CO₂ feed is supplied to the shell space of the HP carbamate condenser shell space. This may be advantageously used to correct the N/C ratio for optimum carbamate condensation, in particular to decrease the N/C ratio, for instance to increase the condensation temperature. In an interesting embodiment, a part of the CO₂ feed is supplied directly to the shell space of the HP carbamate condenser.

Optionally, a part of the CO₂ feed is supplied to the HP stripper, e.g. to the tube side bottom, and thereby indirectly to the HP carbamate condenser. The CO₂ feed may comprise some O₂ and thereby provide for passivation of the HP thermal stripper.

For example up to 50 mol% of the (fresh) CO₂ feed is supplied to the stripper, e.g. at least 1 mol.%, for instance up to 20 mol.% or up to 15 mol.% (or wt.%), for example 10-30 mol.%. For example up to 80 mol.% of the CO₂ feed is supplied (directly) to the condenser, e.g. at least 1 mol.%, preferably up to 40 mol.%. Preferably at least 50 mol.% of the CO₂ feed is supplied directly to the reactor. All said percentages can also refer to wt.%. Herein, amounts CO₂ are percentages relative to the CO₂ feed received at battery limit of the urea plant, e.g. from an ammonia plant, such as from the synthesis gas production section of an ammonia plant. In an advantageous embodiment, especially in case of a revamp, the additional CO₂ is supplied directly to the HP carbamate condenser shell space.

The inventive urea production process involves reacting NH₃ and CO₂ in the reactor giving a urea synthesis stream; supplying at least a liquid fraction of the synthesis stream to the stripper, optionally providing the entire urea synthesis stream to the stripper; supplying gas from the stripper, i.e. from the tubes, to the shell space of the HP carbamate condenser; and condensing said gas in said shell space of the HP carbamate condenser, such that carbamate condensation is carried out in the shell space. The process is carried out in the plant as described.

The liquid outlet temperature of the condenser is e.g. at least 165°C, or at least 170°C, and e.g. up to 180°C. This relatively high liquid outlet temperature is advantageously provided by the higher condensation temperature due to the different composition of the shell side process medium in the HP carbamate condenser compared to process medium in a kettle-type carbamate condenser, and may advantageously also contribute to a higher temperature in the reactor. This in turn could be used for achieving a higher conversion in the reactor, a relatively smaller reactor, and/or a lower operating pressure of the reactor, or a combination of these. A lower reactor pressure may in turn provide for lower pressure and advantageously increased stripping efficiency in the HP stripper and/or lower steam consumption in the HP stripper. All preferences for the plant apply equally for the urea production process.

The invention also pertains to a method of modifying an existing urea production plant to obtain a modified urea production plant that is a urea production plant according to the invention. The existing urea production plant comprises a reactor, a thermal stripper, and a kettle-type high pressure (HP) carbamate condenser, all in the HP synthesis section. The kettle-type HP carbamate condenser is a shell-and-tube heat exchanger configured for receiving gas from the thermal stripper in the tubes, i.e. on the tube side of the kettle-type HP carbamate condenser. The method involves a step of replacing the kettle-type HP carbamate condenser by a replacement high pressure carbamate condenser that is a shell-and-tube heat exchanger comprising a tube bundle and a shell space having a gas inlet. The replacement HP carbamate condenser is configured for receiving gas from the stripper in the shell space and of condensation of said gas into carbamate in said shell space. The replacement HP carbamate condenser is for instance a pool condenser or a pool reactor. All features and preferences described herein for the HP carbamate condenser of the inventive urea plant apply also for the replacement HP carbamate condenser. The method further involves connecting a gas outlet of the thermal stripper with said gas inlet of the shell space. Preferably the method also involves providing a flow line for supplying a part of the CO₂ feed to the shell space. All preferences described for the inventive urea plant also apply for the modified urea production plant. The method optionally also involves adding a stream drum as discussed hereinabove.

Very elegantly, the method of the present invention involves replacing a kettle-type HP condenser in an existing urea plant of the thermal stripping type with a submerged condenser, i.e. a shell-and-tube condenser with condensation in the shell side, can advantageously generate e.g. up to 60% additional reaction volume, such as e.g. 50 - 60% additional reaction volume. This additional reaction volume can be used for providing additional plant capacity or decreased energy consumption (higher pressure steam raised in the condenser), or a combination of both. For instance, a capacity increase of e.g. 1 - 5 % can be obtained by replacement of a kettle-type HP condenser with a HP carbamate condenser as used in the present invention.

Moreover, these advantages can be obtained with low capital expenditure and without a need for significant changes in the HP synthesis section.

The existing plant comprises a HP stripper of the thermal stripping type and may optionally comprise further HP strippers, e.g. of the CO₂ stripping type, in series or in parallel.

The method of modifying an existing plant can be used as a revamp of the existing plant. Advantageously, only minimum adjustments in the HP synthesis section are necessary.

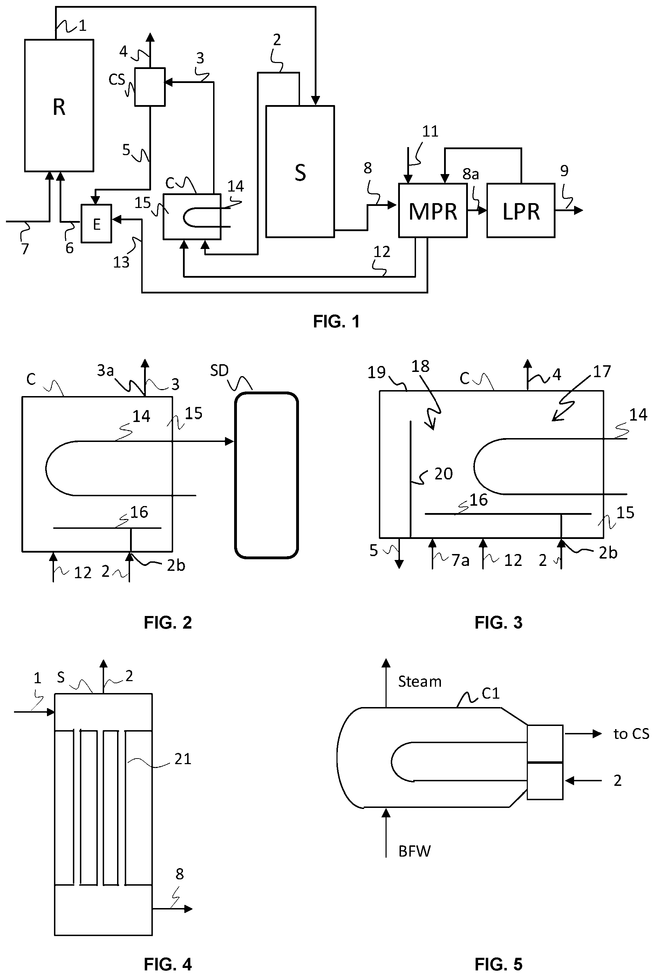

Fig. 1 illustrates an example process scheme (plant and process) according to the invention. The urea plant comprises a HP synthesis section comprising the reactor (R), the stripper (S), and the condenser (C). The HP carbamate condenser (C) is a shell-and-tube heat exchanger with a horizontal U-shaped tube bundle (14) and a shell space (15). The urea synthesis stream (1) from the reactor (R) is supplied to the top of the stripper (S), which is a thermal stripper. The gas stream (2) from the stripper comprises CO₂ and NH₃ is supplied to the shell space (15) of the condenser (C). Steam is raised in the tubes (14) from water, e.g. from boiler feed water. A liquid phase comprising ammonium carbamate is formed in the shell space (15) and the carbamate is already partly reacted into urea and water in the shell space (15). The liquid (3) from the shell space (15) is supplied, together with gaseous components from shell space (15) of the condenser (C), to the carbamate separator (CS) which is a gas/liquid separator operating at HP. Gas (4) from the carbamate separator is supplied e.g. to the MP recovery section (MPR). The liquid (5) from the carbamate separator (CS) is supplied to the ejector (E). The fluid (6) from the ejector (E) is supplied to the reactor (R) which in the illustrated example also receives all of the CO₂ feed (7). In other example embodiments, as illustrated in Fig. 3, a part (7a) of the CO₂ feed (7) is supplied to the HP carbamate condenser (C). Stripped urea solution (8) from the HP stripper (S) is supplied to the MP recovery section (MPR). Stripped urea solution (8a) from the MP recovery section (MPR) is supplied to the LP recovery section (LPR) to give urea solution (9) which is supplied e.g. to an evaporation section (not shown). Carbamate solution (10) from the LP recovery section (LPR) is supplied to the MP recovery section (MPR). The MP recovery section (MPR) also receives the NH₃ feed (11). A MP carbamate recycle solution (12) is supplied, using a HP carbamate pump (not shown), from the MP recovery section (MPR) to the HP synthesis section, in particular to the shell side space (15) of the HP carbamate condenser (C). A separate stream (13) of NH₃ including the feed NH₃ and recycle of NH₃ is supplied, using a HP ammonia pump (not shown), from the MP recovery section (MPR) to the HP ejector (E) as motive fluid.

**Fig. 2** schematically illustrates an example HP carbamate condenser (C) which can be used in the inventive process, plant and method, e.g. in the process scheme of Fig. 1. The HP carbamate condenser (C) is in particular a pool condenser. The condenser comprises a U-shaped tube bundle (14) and a sparger (16) for distributing gas (2) from the stripper (S) into the shell space (15). The shell space (15) also comprises a gas inlet (2b) for receiving said gas (2) from the stripper (S). The condenser (C) comprises a single outlet (3a) from the shell space through which a fluid stream (3) containing both liquid and gas is withdrawn. The outlets of the tubes (14) are connected to a steam drum (SD). The stream drum may be directly attached to the condenser vessel. The MP carbamate recycle solution (12) is supplied to the shell space (15) of the HP carbamate condenser (C).

**Fig. 3** schematically illustrates an example HP carbamate condenser (C) which can be used in the inventive process, plant and method, e.g. in the process scheme of Fig. 1. In particular a pool reactor is illustrated as HP carbamate condenser (C) . The condenser (C) comprises a U-shaped tube bundle (14) and a sparger (16) for distributing gas (2) from the stripper (S) into the shell space (15). The condenser (C) comprises the shell space (15) a gas inlet (2b) for gas (2) from the stripper (S), a gas outlet (4), and a separate liquid outlet (5). The shell space (15) comprises a condensation zone (17) around the U-shaped tube bundle (14), and a reaction zone (18) located beyond the tube bundle, between the vessel wall (19) and the bend of the U-shaped tube bundle (14). The condenser (C) comprises a shell or vessel wall (19) which in operation is in contact with corrosive process medium, in particular carbamate, on the inside and is provided e.g. with a corrosion resistant lining on the inside. The sparger (16) extends horizontally in both the condensation zone (17) and the reaction zone (18). In this way, condensation of gas from the part of the sparger (16) which extends into the reaction zone (18) may provide for heat for the urea formation reaction. A baffle (20) acts as an overflow weir and thereby provides gas/liquid separation. The top of baffle (20) is arranged higher than the top of the tube bundle (14). In this way the tube bundle (14) is submerged in operation. The baffle (20) is horizontally spaced from the bend of the U-shaped tube bundle (14) by e.g. at least 10%, e.g. up to 50%, of the shell space (15) horizontal length to provide for residence time in the reaction zone (18) located between that overflow weir baffle (20) and said bend of the U-shaped tube bundle (14). The MP carbamate recycle solution (12) is supplied to the shell space (15) of the HP carbamate condenser (C). The shell space (15) has an inlet for a part (7a) of the CO₂ feed supplied directly to the shell space (15) in this embodiment. The outlets of the tubes (14) can be connected to a steam drum (not shown).

**Fig. 4** schematically illustrates an example HP stripper (S) as can be used in the inventive process and plant, e.g. in the process scheme of Fig. 1. The stripper (S) has a top inlet for a urea synthesis stream or solution (1), a top outlet for gas (2), a bottom outlet for stripped urea solution (8) and comprises stripper tubes (21). In practice the stripper (S) comprises numerous stripper tubes (21), e.g. more than 1000. The stripper (S) receives steam on the shell side for heating the urea solution (1) which solution is provided as a falling film in the tubes (21) to cause the stripping in order to remove ammonia and carbamate from the urea solution (1).

**Fig. 5** schematically illustrates a kettle-type boiler (C1) as used in existing urea plants of the thermal-stripping type. Gas (2) from the stripper is supplied to the tubes of the U-shaped tube bundle. The tube bundle outlet is connected to the carbamate separator (CS). Steam is raised on the shell side from boiler feed water (BFW).

In this application, for process streams (i.e. not for steam lines), high pressure (HP) is above 100 bar, for instance 120 to 300 bar, typically 150 to 200 bar. Medium pressure (MP) is for example 10 to 70 bar (including intermediate pressure of 30 to 70 bar), in particular 10 to 40 bar, and low pressure (LP) is for example 1.0 to 10 bar, in particular 1.0 to 8 bar, e.g. 1.5 to 5 bar. All pressures are in bar absolute. The N/C ratio as used herein for urea solutions and carbamate streams reflects the composition of the so-called initial mixture before urea production, consisting only of NH₃, CO₂ and H₂O, as used in the art of urea plants, and is the molar ratio. The N/C ratio for gas streams indicates the molar ratio of NH₃ to CO₂.

The term 'carbamate' as used herein refers to ammonium carbamate, as the term is used in the art of urea production. Condensation in a carbamate condenser refers to so-called carbamate condensation, which involves the reaction of NH₃ and CO₂ into ammonium carbamate, such that in effect gaseous NH₃ and CO₂ become carbamate which is included in a liquid phase. Carbamate decomposition refers to the dissociation reaction of carbamate into NH₃ and CO₂.

The terms 'typical' and 'in particular' are used to indicate features that can be used in some embodiments but that are not mandatory. Preferred features are not mandatory.

The term 'comprising' and derived forms are used to indicate that further elements and steps than those recited can be present.

As used herein, the terms shell side, shell side space, and shell space generally refer to the space between the tubes and confined by the shell (vessel wall) of the a shell-and-tube heat exchanger. The term 'tube side' refers to the internal space of the tubes.

The disclosure pertains to a urea plant and process of the thermal stripping type as well as to a method of modifying a urea plant of the thermal stripping type. Described is a urea plant with a thermal stripper and a high pressure carbamate condenser with a shell space wherein gas from the stripper is condensed in the shell space.

The invention will now be further illustrated by the following example, which does not limit the claims.

### Example 1

In a thermal-stripping (self-stripping) type urea plant, the existing kettle-type HP carbamate condenser was replaced by a pool condenser. The results are shown in Table 1; liquid outlet refers to the outlet of the process side of the condenser. The two designs are compared on the basis of equal amount of off-gas from the process side of the condenser (e.g. off-gas supplied to the MP recovery section).

The liquid outlet temperature is advantageously significantly higher in the inventive plant due to the higher concentration of urea.

The higher temperature in the process side of the condenser can be used e.g. for raising higher pressure steam which was found to provide for a reduction in steam consumption of the plant of 0-10%, typically 2 - 5 %. The 17 wt.% urea content of the liquid at the outlet of the condenser can be used to increase the capacity of the urea plant by 0-7 %, typically between 2-5 % in this example.

**Table 1**

| | **Reference** | **Inventive** |
|---|---|---|
| HP Condenser type | Kettle type | Pool condenser |
| Off gas amount from condenser | 6000 kg/hr | 6000 kg/hr |
| Liquid outlet temperature | 160°C | 172°C |
| Liquid outlet urea content | 0 wt.% | 17 wt.% |

## Claims

1. A urea production plant comprising a high pressure (HP) synthesis section comprising a reactor (R), a thermal stripper (S), and a high pressure carbamate condenser (C), wherein the high pressure carbamate condenser (C) comprises a shell-and-tube heat exchanger comprising a tube bundle (14) and a shell space (15), wherein the plant comprises a gas flow line (2) from a gas outlet (2a) of the thermal stripper (S) to a gas inlet (2b) of said shell space (15); wherein the plant further comprises a medium pressure (MP) recovery section (MPR), wherein the MP recovery section (MPR) has a carbamate recycle flow line (12) and a separate ammonia recycle flow line (13) to the HP synthesis section.

2. A urea production plant according to claim 1, wherein the condenser (C) comprises a sparger (16) in the shell space (15) for distributing gas (2) from the thermal stripper (S) into the shell space (15).

3. A urea production plant according to claim 1 or 2, wherein the tube bundle (14) of the condenser (C) is a horizontal tube bundle, preferably a U-shaped tube bundle.

4. A urea production plant according to any of the preceding claims, the plant further comprising a steam drum (SD) connected to an outlet of the tube bundle (14) of the condenser (C).

5. A urea production plant according to any of the preceding claims, wherein the thermal stripper (S) comprises stripper tubes (21), wherein the stripper tubes are made of Zr or Ti or are bimetallic stripper tubes; wherein the bimetallic stripper tubes preferably comprise an external tube made of steel and an internal tube made of e.g. zirconium.

6. A urea production plant according to any of the preceding claims, wherein the shell space (15) of the condenser (C) comprises an outlet (3a) for fluid comprising liquid and wherein the plant comprises a liquid flow connection (3) from said outlet (3a) to the reactor (R).

7. A urea production plant according to any of the preceding claims, wherein the condenser (C) is a vessel comprising a horizontal U-shaped tube bundle (14),
a condensation zone (17) and a reaction zone (18).

8. A urea production plant according to any of the preceding claims, wherein the condenser (C) comprises a shell (19), wherein the shell (19) is provided with a lining of urea grade corrosion resistant steel, e.g. with a lining in duplex ferritic-austenitic stainless steel.

9. A urea production plant according to claim 1, comprising a flow line for supplying a part of a CO₂ feed of the plant to the HP carbamate condenser, preferably directly and/or through the HP stripper.

10. A urea production plant according to any of the preceding claims, wherein
A) the HP synthesis section comprises a carbamate separator and an ejector, and wherein the shell space of the high pressure carbamate condenser (C) comprises an outlet for a fluid stream containing both gas and liquid, which outlet is connected to said carbamate separator being a high pressure gas/liquid separator having a gas outlet and a liquid outlet, wherein the liquid outlet of the separator is connected through the ejector to an inlet of the reactor, to supply a liquid containing carbamate, urea and water to the reactor, and the gas outlet is connected with the MP recovery section;
or
B) the high pressure carbamate condenser (C) has a gas outlet and a separate liquid outlet both from the shell space, wherein said liquid outlet is connected indirectly with the reactor through a high pressure (HP) ejector.

11. A urea production process carried out in a urea production plant according to any of the preceding claims, the process comprising:
- reacting NH₃ feed and CO₂ feed in the reactor (R) giving a urea synthesis stream (1);
- supplying at least a liquid part of the synthesis stream (1) to the stripper (S);
- supplying gas (2) from the stripper to the shell space (15) of the HP carbamate condenser (C); and
- condensing said gas (2) in said shell space (15) of the HP carbamate condenser (C).

12. A urea production process carried out according to claim 11, wherein the reactor is operated with a reactor outlet N/C ratio of 3.2 - 3.6; and/or wherein the stripper is operated at a pressure at least 2 bar lower than the reactor.

13. A urea production process carried out according to any of claims 11-12, wherein a liquid (5) is withdrawn from said shell space (15) comprising at least 5 wt.% urea, preferably at least 10 wt.% urea.

14. A urea production process carried out according to any of claims 11-13, wherein the condensation temperature in said shell space (15) is at least 165°C.

15. A urea production process according to any of claims 11 to 14, wherein a part of the CO₂ feed is supplied to the HP carbamate condenser.

16. A urea production process according to claim 15, wherein a part, preferably 1 mol.% to 50 mol.%, of the CO₂ feed is supplied to the HP carbamate condenser through the HP stripper.

17. A urea production process according to claim 15 or 16, wherein a part, preferably 1 mol.% to 80 mol.%, of the CO₂ feed is supplied directly to the HP carbamate condenser.

18. A urea production process according to claim any of claims 15 to 17, wherein at least 50 mol.% of the CO₂ feed is supplied directly to the reactor.

19. A urea production process according to claim any of claims 11-18, wherein the NH₃ content of the bottom effluent of the stripper is 20 - 30 wt.% and the stripper outlet temperature is at least 200°C.

20. A method of modifying an existing urea production plant, the existing urea production plant comprising a reactor (R), a thermal stripper (S), and a kettle-type high pressure carbamate condenser (C1),
wherein the kettle-type high pressure carbamate condenser (C1) is a shell-and-tube heat exchanger configured for receiving gas from the thermal stripper (S) in the tubes; the method involving:
- replacing said kettle-type HP carbamate condenser (C1) by a replacement high pressure carbamate condenser (C) that is a shell-and-tube heat exchanger comprising a tube bundle (14) and a shell space (15) having a gas inlet (2b) and configured for receiving gas (2) from the stripper and condensation of said gas into carbamate in said shell space (15); and
- connecting a gas outlet (2a) of the thermal stripper (S) with said gas inlet (2b) of the shell space (15),
wherein the method results in a urea production plant according to claim 1.

21. The method according to claim 20, wherein the method comprises: providing a flow line for a CO₂ feed to the shell space (15), preferably wherein this flow line is a flow line to supply CO₂ directly to the shell space (15) of the replacement high pressure carbamate condenser.

22. The method according to claim 20 or 21, wherein
A) the HP synthesis section comprises carbamate separator and an ejector, and the shell space of the high pressure carbamate condenser (C) comprises an outlet for a fluid stream containing both gas and liquid, which outlet is connected to said carbamate separator being a high pressure gas/liquid separator having a gas outlet and a liquid outlet, wherein the liquid outlet of the separator is connected through the ejector to an inlet of the reactor, to supply a liquid containing carbamate, urea and water to the reactor, and the gas outlet is connected with the MP recovery section;
or
B) the high pressure carbamate condenser (C) has a gas outlet and a separate liquid outlet both from the shell space, wherein said liquid outlet is connected indirectly with the reactor through a high pressure (HP) ejector.

## Patentansprüche

1. Harnstoffherstellungsanlage, umfassend einen Hochdruck(HP)-Syntheseabschnitt, umfassend einen Reaktor (R), einen thermischen Stripper (S) und einen Hochdruck-Carbamatkondensator (C), wobei der Hochdruck-Carbamatkondensator (C) einen Röhrenkesselwärmetauscher umfasst, umfassend ein Rohrbündel (14) und einen Mantelraum (15), wobei die Anlage eine Gasstromleitung (2) von einem Gasauslass (2a) des thermischen Strippers (S) zu einem Gaseinlass (2b) des genannten Mantelraums (15) umfasst; wobei die Anlage ferner einen Mitteldruck(MP)-Rückgewinnungsabschnitt (MPR) umfasst, wobei der MP-Rückgewinnungsabschnitt (MPR) eine Carbamat-Rückführungsstromleitung (12) und eine separate Ammoniak-Rückführungsstromleitung (13) zum HP-Syntheseabschnitt hat.

2. Harnstoffherstellungsanlage nach Anspruch 1, wobei der Kondensator (C) einen Verteiler (16) im Mantelraum (15) zum Verteilen von Gas (2) vom thermischen Stripper (S) in den Mantelraum (15) umfasst.

3. Harnstoffherstellungsanlage nach Anspruch 1 oder 2, wobei das Rohrbündel (14) des Kondensators (C) ein horizontales Rohrbündel, vorzugsweise ein U-förmiges Rohrbündel, ist.

4. Harnstoffherstellungsanlage nach einem der vorhergehenden Ansprüche, wobei die Anlage ferner eine Dampftrommel (SD), verbunden mit einem Auslass des Rohrbündels (14) des Kondensators (C), umfasst.

5. Harnstoffherstellungsanlage nach einem der vorhergehenden Ansprüche, wobei der thermische Stripper (S) Stripperrohre (21) umfasst, wobei die Stripperrohre aus Zr oder Ti hergestellt sind oder bimetallische Stripperrohre sind; wobei die bimetallischen Stripperrohre vorzugsweise ein Außenrohr hergestellt aus Stahl und ein Innenrohr hergestellt aus z. B. Zirkonium umfassen.

6. Harnstoffherstellungsanlage nach einem der vorhergehenden Ansprüche, wobei der Mantelraum (15) des Kondensators (C) einen Auslass (3a) für Fluid umfasst, umfassend Flüssigkeit, und wobei die Anlage eine Flüssigkeitsstromverbindung (3) von dem Auslass (3a) zum Reaktor (R) umfasst.

7. Harnstoffherstellungsanlage nach einem der vorhergehenden Ansprüche, wobei der Kondensator (C) ein Behälter ist, umfassend ein horizontales U-förmiges Rohrbündel (14), eine Kondensationszone (17) und eine Reaktionszone (18).

8. Harnstoffherstellungsanlage nach einem der vorhergehenden Ansprüche, wobei der Kondensator (C) einen Mantel (19) umfasst, wobei der Mantel (19) mit einer Auskleidung aus für Harnstoff geeignetem, korrosionsbeständigem Stahl versehen ist, z. B. mit einer doppelten Auskleidung aus ferritisch-austenitischem rostfreiem Stahl.

9. Harnstoffherstellungsanlage nach Anspruch 1, umfassend eine Strömungsleitung zum Zuführen eines Teils eines CO₂-Einsatzmaterials der Anlage zum HP-Carbamatkondensator, vorzugsweise direkt und/oder durch den HP-Stripper.

10. Harnstoffherstellungsanlage nach einem der vorhergehenden Ansprüche, wobei:
A) der HP-Syntheseabschnitt einen Carbamatabscheider und einen Ejektor umfasst, und wobei der Mantelraum des Hochdruck-Carbamatkondensators (C) einen Auslass für einen Fluidstrom umfasst, der sowohl Gas als auch Flüssigkeit enthält, wobei dieser Auslass mit dem Carbamatabscheider verbunden ist, der ein Hochdruck-Gas-/Flüssigkeitsabscheider ist, mit einem Gasauslass und einem Flüssigkeitsauslass, wobei der Flüssigkeitsauslass des Abscheiders durch den Ejektor mit einem Einlass des Reaktors verbunden ist, um dem Reaktor eine Flüssigkeit zuzuführen, die Carbamat, Harnstoff und Wasser enthält, und der Gasauslass mit dem MP-Rückgewinnungsabschnitt verbunden ist;
oder
B) der Hochdruck-Carbamatkondensator (C) einen Gasauslass und einen separaten Flüssigkeitsauslass, beide vom Mantelraum, hat, wobei der Flüssigkeitsauslass über einen Hochdruck(HP)-Ejektor indirekt mit dem Reaktor verbunden ist.

11. Harnstoffherstellungsverfahren, ausgeführt in einer Harnstoffherstellungsanlage nach einem der vorhergehenden Ansprüche, wobei das Verfahren umfasst:
- Reagieren des NH₃-Einsatzmaterials und des CO₂-Einsatzmaterials im Reaktor (R), das ein Harnstoffsynthesestrom (1) ergibt;
- Zuführen wenigstens eines flüssigen Teils des Synthesestroms (1) zum Stripper (S);
- Zuführen von Gas (2) vom Stripper zum Mantelraum (15) des HP-Carbamatkondensators (C); und
- Kondensieren des Gases (2) in dem Mantelraum (15) des HP-Carbamatkondensators (C).

12. Harnstoffherstellungsverfahren, ausgeführt nach Anspruch 11, wobei der Reaktor mit einem N/C-Verhältnis am Reaktorauslass von 3,2- 3,6 betrieben wird; und/oder wobei der Stripper mit einem Druck betrieben wird, der mindestens 2 bar niedriger ist als der Reaktor.

13. Harnstoffherstellungsverfahren, ausgeführt nach einem der Ansprüche 11 bis 12, wobei aus dem Mantelraum (15) eine Flüssigkeit (5) abgezogen wird, umfassend mindestens 5 Gew.-% Harnstoff, vorzugsweise mindestens 10 Gew.-% Harnstoff.

14. Harnstoffherstellungsverfahren, ausgeführt nach einem der Ansprüche 11 bis 13, wobei die Kondensationstemperatur in dem Mantelraum (15) mindestens 165 °C ist.

15. Harnstoffherstellungsverfahren nach einem der Ansprüche 11 bis 14, wobei ein Teil des CO₂ -Einsatzmaterials dem HP-Carbamatkondensator zugeführt wird.

16. Harnstoffherstellungsverfahren nach Anspruch 15, wobei ein Teil, vorzugsweise 1 Molprozent bis 50 Molprozent, des CO₂ - Einsatzmaterials dem HP-Carbamatkondensator über den HP-Stripper zugeführt wird.

17. Harnstoffherstellungsverfahren nach Anspruch 15 oder 16, wobei ein Teil, vorzugsweise 1 Molprozent bis 80 Molprozent, des CO₂ - Einsatzmaterials direkt dem HP-Carbamatkondensator zugeführt wird.

18. Harnstoffherstellungsverfahren nach einem der Ansprüche 15 bis 17, wobei mindestens 50 Molprozent des CO₂-Einsatzmaterials direkt dem Reaktor zugeführt werden.

19. Harnstoffherstellungsverfahren nach einem der Ansprüche 11 bis 18, wobei der NH₃-Gehalt der am Boden des Strippers abfließenden Flüssigkeit 20-30 Gew.-% ist und die Stripperauslasstemperatur mindestens 200 °C ist.

20. Verfahren zum Modifizieren einer bestehenden Harnstoffherstellungsanlage, wobei die bestehende Harnstoffherstellungsanlage einen Reaktor (R), einen thermischen Stripper (S) und einen Hochdruck-Carbamatkondensator (C1) vom Kesseltyp umfasst,
wobei der Hochdruck-Carbamatkondensator (C1) vom Kesseltyp ein Röhrenkesselwärmetauscher ist, der zum Aufnehmen von Gas vom thermischen Stripper (S) in den Rohren konfiguriert ist; wobei das Verfahren involviert:
- Ersetzen des HP-Carbamatkondensators (C1) vom Kesseltyp durch einen Ersatz-Hochdruck-Carbamatkondensator (C), der ein Röhrenkesselwärmetauscher ist, umfassend ein Rohrbündel (14) und einen Mantelraum (15) mit einem Gaseinlass (2b) und konfiguriert zum Aufnehmen von Gas (2) vom Stripper und Kondensation des Gases in dem Mantelraum (15) in Carbamat; und
- Verbinden eines Gasauslasses (2a) des thermischen Strippers (S) mit dem Gaseinlass (2b) des Mantelraums (15),
wobei das Verfahren in einer Harnstoffherstellungsanlage nach Anspruch 1 resultiert.

21. Verfahren nach Anspruch 20, wobei das Verfahren umfasst: Bereitstellen einer Strömungsleitung für ein CO₂-Einsatzmaterial zum Mantelraum (15), wobei diese Strömungsleitung vorzugsweise eine Strömungsleitung zum direkten Zuführen von CO₂ in den Mantelraum (15) des Ersatz-Hochdruck-Carbamatkondensators ist.

22. Verfahren nach Anspruch 20 oder 21, wobei
A) der HP-Syntheseabschnitt einen Carbamatabscheider und einen Ejektor umfasst, und der Mantelraum des Hochdruck-Carbamatkondensators (C) einen Auslass für einen Fluidstrom umfasst, der sowohl Gas als auch Flüssigkeit enthält, wobei der Auslass mit dem Carbamatabscheider verbunden ist, der ein Hochdruck-Gas-/Flüssigkeitsabscheider ist, mit einem Gasauslass und einem Flüssigkeitsauslass, wobei der Flüssigkeitsauslass des Abscheiders über den Ejektor mit einem Einlass des Reaktors verbunden ist, um dem Reaktor eine carbamathaltige Flüssigkeit, Harnstoff und Wasser zuzuführen, und der Gasauslass mit dem MP-Rückgewinnungsabschnitt verbunden ist;
oder
B) der Hochdruck-Carbamatkondensator (C) einen Gasauslass und einen separaten Flüssigkeitsauslass, beide vom Mantelraum, hat, wobei der Flüssigkeitsauslass über einen Hochdruck(HP)-Ejektor indirekt mit dem Reaktor verbunden ist.

## Revendications

1. Unité de production d'urée comprenant une section de synthèse haute pression (HP) comprenant un réacteur (R), un désorbeur thermique (S), et un condenseur de carbamate haute pression (C), dans laquelle le condenseur de carbamate haute pression (C) comprend un échangeur de chaleur à calandre et tubes comprenant un faisceau de tubes (14) et un espace de calandre (15), laquelle unité comprend une ligne d'écoulement de gaz (2) allant d'une sortie de gaz (2a) du désorbeur thermique (S) à une entrée de gaz (2b) dudit espace de calandre (15) ; laquelle unité comprend en outre une section de récupération moyenne pression (MP) (MPR), dans laquelle la section de récupération MP (MPR) a une ligne d'écoulement de recyclage de carbamate (12) et une ligne d'écoulement de recyclage d'ammoniac (13) séparée allant à la section de synthèse HP.

2. Unité de production d'urée selon la revendication 1, dans laquelle le condenseur (C) comprend un diffuseur (16) dans l'espace de calandre (15) pour distribuer un gaz (2) provenant du désorbeur thermique (S) dans l'espace de calandre (15).

3. Unité de production d'urée selon la revendication 1 ou 2, dans laquelle le faisceau de tubes (14) du condenseur (C) est un faisceau de tubes horizontal, de préférence un faisceau de tubes en forme de U.

4. Unité de production d'urée selon l'une quelconque des revendications précédentes, l'unité comprenant en outre un collecteur de vapeur (SD) connecté à une sortie du faisceau de tubes (14) du condenseur (C).

5. Unité de production d'urée selon l'une quelconque des revendications précédentes, dans laquelle le désorbeur thermique (S) comprend des tubes de désorbeur (21), dans laquelle les tubes de désorbeur sont faits en Zr ou Ti ou sont des tubes de désorbeur bimétalliques ; dans laquelle les tubes de désorbeur bimétalliques comprennent de préférence un tube externe fait en acier et un tube interne fait par exemple en zirconium.

6. Unité de production d'urée selon l'une quelconque des revendications précédentes, dans laquelle l'espace de calandre (15) du condenseur (C) comprend une sortie (3a) pour fluide comprenant du liquide, et laquelle unité comprend une connexion d'écoulement de liquide (3) allant de ladite sortie (3a) au réacteur (R).

7. Unité de production d'urée selon l'une quelconque des revendications précédentes, dans laquelle le condenseur (C) est une cuve comprenant un faisceau de tubes en forme de U horizontal (14), une zone de condensation (17), et une zone de réaction (18).

8. Unité de production d'urée selon l'une quelconque des revendications précédentes, dans laquelle le condenseur (C) comprend une calandre (19), dans laquelle la calandre (19) est dotée d'un chemisage en acier résistant à la corrosion de qualité pour urée, par exemple d'un chemisage en acier inoxydable duplex ferritique austénitique.

9. Unité de production d'urée selon la revendication 1, comprenant une ligne d'écoulement pour délivrer une partie d'une charge de CO₂ de l'unité au condenseur de carbamate HP, de préférence directement et/ou par l'intermédiaire du désorbeur HP.

10. Unité de production d'urée selon l'une quelconque des revendications précédentes, dans laquelle
A) la section de synthèse HP comprend un séparateur de carbamate et un éjecteur, et dans laquelle l'espace de calandre du condenseur de carbamate haute pression (C) comprend une sortie pour un courant de fluide contenant à la fois du gaz et du liquide, laquelle sortie est connectée audit séparateur de carbamate qui est un séparateur de gaz/liquide haute pression ayant une sortie de gaz et une sortie de liquide, dans laquelle la sortie de liquide du séparateur est connectée par l'intermédiaire de l'éjecteur à une entrée du réacteur, pour délivrer au réacteur un liquide contenant du carbamate, de l'urée et de l'eau, et la sortie de gaz est connectée à la section de récupération MP ;
ou
B) le condenseur de carbamate haute pression (C) a une sortie de gaz et une sortie de liquide séparées partant toutes deux de l'espace de calandre, dans lequel ladite sortie de liquide est connectée indirectement au réacteur par l'intermédiaire d'un éjecteur haute pression (HP).

11. Procédé de production d'urée mis en œuvre dans une unité de production d'urée selon l'une quelconque des revendications précédentes, le procédé comprenant :
- la réaction d'une charge de NH₃ et d'une charge de CO₂ dans le réacteur (R), ce qui donne un courant de synthèse d'urée (1) ;
- la fourniture d'au moins une partie liquide du courant de synthèse (1) au désorbeur (S) ;
- la fourniture de gaz (2) provenant du désorbeur à l'espace de calandre (15) du condenseur de carbamate HP (C) ; et
- la condensation dudit gaz (2) dans ledit espace de calandre (15) du condenseur de carbamate HP (C).

12. Procédé de production d'urée mis en œuvre selon la revendication 11, dans lequel le réacteur fonctionne avec un rapport N/C de sortie de réacteur de 3,2 à 3,6 ; et/ou dans lequel le désorbeur fonctionne sous une pression inférieure d'au moins 2 bar à celle du réacteur.

13. Procédé de production d'urée mis en œuvre selon l'une quelconque des revendications 11 et 12, dans lequel un liquide (5) comprenant au moins 5 % en poids d'urée, de préférence au moins 10 % en poids d'urée, est soutiré dudit espace de calandre (15).

14. Procédé de production d'urée mis en œuvre selon l'une quelconque des revendications 11 à 13, dans lequel la température de condensation dans ledit espace de calandre (15) est d'au moins 165°C.

15. Procédé de production d'urée selon l'une quelconque des revendications 11 à 14, dans lequel une partie de la charge de CO₂ est fournie au condenseur de carbamate HP.

16. Procédé de production d'urée selon la revendication 15, dans lequel une partie, de préférence 1 % en moles à 50 % en moles, de la charge de CO₂, sont fournis au condenseur de carbamate HP par l'intermédiaire du désorbeur HP.

17. Procédé de production d'urée selon la revendication 15 ou 16, dans lequel une partie, de préférence 1 % en moles à 80 % en moles, de la charge de CO₂ sont fournis directement au condenseur de carbamate HP.

18. Procédé de production d'urée selon l'une quelconque des revendications 15 à 17, dans lequel au moins 50 % en moles de la charge de CO₂ sont fournis directement au réacteur.

19. Procédé de production d'urée selon l'une quelconque des revendications 11 à 18, dans lequel la teneur en NH₃ de l'effluent de fond du désorbeur est de 20 à 30 % en poids et la température de sortie de désorbeur est d'au moins 200°C.

20. Méthode de modification d'une unité de production d'urée existante, l'unité de production d'urée existante comprenant un réacteur (R), un désorbeur thermique (S), et un condensateur de carbamate haute pression de type bouilleur (C1),
dans laquelle le condenseur de carbamate haute pression de type bouilleur (C1) est un échangeur de chaleur à calandre et tubes configuré pour recevoir du gaz depuis le désorbeur thermique (S) dans les tubes ;
la méthode impliquant :
- le remplacement dudit condenseur de carbamate HP de type bouilleur (C1) par un condenseur de carbamate haute pression de remplacement (C) qui est un échangeur de chaleur à calandre et tubes comprenant un faisceau de tubes (14) et un espace de calandre (15) ayant une entrée de gaz (2b) et configuré pour recevoir du gaz (2) depuis le désorbeur et la condensation dudit gaz en carbamate dans ledit espace de calandre (15) ; et
- la connexion d'une sortie de gaz (2a) du désorbeur thermique (S) avec ladite entrée de gaz (2b) de l'espace de calandre (15),
laquelle méthode a pour résultat une unité de production d'urée selon la revendication 1.

21. Méthode selon la revendication 20, laquelle méthode comprend : la fourniture d'une ligne d'écoulement pour une charge de CO₂ vers l'espace de calandre (15), de préférence dans laquelle cette ligne d'écoulement est une ligne d'écoulement pour fournir du CO₂ directement à l'espace de calandre (15) du condenseur de carbamate haute pression de remplacement.

22. Méthode selon la revendication 20 ou 21, dans laquelle
A) la section de synthèse HP comprend un séparateur de carbamate et un éjecteur, et l'espace de calandre du condenseur de carbamate haute pression (C) comprend une sortie pour un courant de fluide contenant à la fois du gaz et du liquide, laquelle sortie est connectée audit séparateur de carbamate qui est un séparateur de gaz/liquide haute pression ayant une sortie de gaz et une sortie de liquide, dans laquelle la sortie de liquide du séparateur est connectée par l'intermédiaire de l'éjecteur à une entrée du réacteur, pour délivrer au réacteur un liquide contenant du carbamate, de l'urée et de l'eau, et la sortie de gaz est connectée à la section de récupération MP ;
ou
B) le condenseur de carbamate haute pression (C) a une sortie de gaz et une sortie de liquide séparées partant toutes deux de l'espace de calandre, dans lequel ladite sortie de liquide est connectée indirectement au réacteur par l'intermédiaire d'un éjecteur haute pression (HP).
